# EUROPÄISCHE PATENTSCHRIFT

(11) **EP 2 677 984 B1**
(45) Veröffentlichungstag und Bekanntmachung des Hinweises auf die Patenterteilung: **25.09.2024**
(21) Anmeldenummer: 12711558.2
(22) Anmeldetag: 21.02.2012
(51) Int. Cl.: A61F 13/551, B32B 27/32, C09J 123/20, C09J 123/08, C09J 123/10, C08L 23/08, C08L 23/20, B32B 27/08, C09J 123/06, C08L 23/06

(54) **EINE GERÄUSCHARM ZU ÖFFNENDE EINSCHLAGVERPACKUNG**
QUIET-OPENING WRAPPER
ENVELOPPE POUVANT ÊTRE OUVERTE AVEC PEU DE BRUIT

(30) Priorität: 23.02.2011 DE 102011012209
(43) Veröffentlichungstag der Anmeldung: 01.01.2014
(73) Patentinhaber: Loparex Germany GmbH & Co. KG, 91301 Forchheim (DE)
(72) Erfinder: SITZMANN, Stefan, 91356 Kirchehrenbach (DE); SCHUHMANN, Michael, 90613 Grosshabersdorf (DE)
(74) Vertreter: Kutzenberger Wolff & Partner
(86) Internationale Anmeldenummer: PCT/EP2012/000748
(87) Internationale Veröffentlichungsnummer: WO 2012/113535

(56) Entgegenhaltungen:
- EP-A1- 0 196 727
- EP-A1- 0 196 727
- EP-A2- 0 435 789
- EP-A2- 0 435 789
- WO-A1-97/48554
- WO-A1-97/48554
- US-A- 4 275 120
- US-A- 4 275 120
- US-A- 4 666 778
- US-A- 4 666 778
- US-A- 4 882 229
- US-A- 4 882 229
- US-A1- 2001 056 270
- US-A1- 2001 056 270

## Beschreibung

Die vorliegende Erfindung betrifft eine geräuscharm zu öffnende Einschlagverpackung (5) für ein flächenförmiges, einseitig mit einem Kleber versehenes Hygieneprodukt (4), die auf einem bahnförmigen Verpackungsmaterial (1) aus einer Kunststofffolie basiert, wobei das beim Öffnen der Einschlagverpackung (5) entstehende Geräusch einem energieäquivalenten Dauerschallpegel LAeq ≤ 60 dB entspricht, gemessen nach der in der Beschreibung angegebenen Methode, **dadurch gekennzeichnet,** dass die Kunststofffolie
a) wenigstens eine heißsiegelbare Schicht bestehend aus einer Mischung aus LDPE und LLDPE, die überwiegend aus LDPE besteht, und aus 3 bis höchstens 30 Gew.-%, bezogen auf das Gesamtgewicht der Schicht a), Polybutylen und ggf. bis zu 20 Gew.-% bezogen auf das Gesamtgewicht der Schicht a) Farbpigmenten und/oder Füllstoffen und
b) ggf. eine Schicht aus Polyethylen mit einer Dichte von 0,88 - 0,94 g/cm³ umfasst
wobei die Schicht a) zumindest in den Abschnitten, die den mit dem Kleber versehenen Bereichen des Hygieneproduktes (4) zugewandt und damit lösbar verbunden sind, eine Release-Beschichtung (2) und im Übrigen zumindest in den zu streifenförmigen Randverschlüssen durch Heißsiegelung verbundenen Teilbereichen (3) der Einschlagverpackung keine Release-Beschichtung aufweist.

Es ist bekannt, dass flächenförmige Hygieneprodukte, insbesondere für Frauen, jeweils gefaltet und einzeln verpackt in Packungen mit unterschiedlicher Stückzahl marktgeführt werden. Für viele Anwender solcher einzeln verpackter Hygieneprodukte stellt das nicht zu überhörende Geräusch beim Öffnen einer solchen Einzelverpackung zur Entnahme des Hygieneprodukts ein ungewolltes, peinliches Ereignis dar, insbesondere wenn man mit der Anwesenheit anderer Menschen, wie z. B. in öffentlichen Toiletten, rechnen muss. Selbst durch eine erhöhte Kraftanstrengung, das Öffnen der Verpackung rascher und damit das peinliche Ereignis kürzer zu gestalten, führt nicht dazu, dass das auffällige Geräusch überhörbar wäre.

EP 0 196 727 A1 offenbart Beutel aus mehrschichtigen Kunststofffolien mit einer Siegelschicht, die in Abhängigkeit von der Siegeltemperatur wiederablösbar gestaltet werden kann. US 4 882 229 A offenbart siegelfähige Einschlagfolien, bei denen die Siegelverbindung sauber wiederablösbar ist. US 4 275 120 A offenbart heißsiegelbare Mehrschichtfolien für Verpackungen, die über einen weiten Temperaturbereich heißsiegelbar sind. US 4 666 778 A offenbart eine heißsiegelbare Verpackungsfolie mit der auch wiederablösbare Siegelverbindungen hergestellt werden können und die gute optische Eigenschaften aufweist. WO 97/48554 A1 offenbart Zusammensetzungen für wiederablösbare Siegelschichten aus einem gleichmäßig verzweigten Ethylen-Polymer und kleinen Mengen von zwei verschiedenen Olefin-Polymeren. US 2001/056270 A1 offenbart Einschlagverpackungen für Hygieneartikel, wobei Kunststofffolien ausdrücklich als ungeeignet angesehen werden.

Andererseits haben Einzelverpackungen, wie Einschlagverpackungen von Hygieneprodukten nicht nur aus hygienischen Gründen eine immer größere Bedeutung erlangt, sondern auch aufgrund ihrer in den meisten Fällen direkt gegebenen Klebefähigkeit, die eine einfache Anwendung erlaubt. Solche einseitig klebefähigen flächenförmigen Hygieneprodukte, wie z. B. Damenbinden, wurden mit immer besser an das klebefähige Hygieneprodukt angepassten Einschlagverpackungen geschützt. Für solche Verpackungen ist es bekannt, Verpackungsmaterialien, wie z. B. Mono- oder Mehrschicht-Kunststofffolien zu verwenden, die bereits einseitig mit einer Anti-Haftbeschichtung, vorzugsweise sogar entsprechend der Anordnung des Klebstoffes, auf der klebefähigen Seite des Hygieneproduktes ausgerüstet sind und darüber hinaus zumindest in den Teilbereichen der Kunststofffolie, die für die Randverschlüsse der Einschlagverpackung benötigt werden, keine Anti-Haftbeschichtung, insbesondere keine Polysiloxan-Beschichtung, aufzuweisen. Diese teilweise Anti-Haftbeschichtung der Folienoberfläche, die der mit Kleber versehenen Seite des Hygieneprodukts zugewandt ist, kann auch streifenförmig in Richtung der Folienbahnen sein, wobei zumindest in den Abschnitten der Folienbahn, aus denen die Randverschlüsse der Einschlagverpackung durch Versiegelung oder Verkrümperung oder Verklebung hergestellt werden, keine Anti-Haftbeschichtung vorliegt. Dadurch gelingt es, ohne besonderen technischen Aufwand, durch übliche Heißversiegelungsverfahren oder durch das aufwendigere Verkrümperungsverfahren die Einschlagverpackungen an den Seitenrändern gut zu verbinden, ohne das sehr wartungsaufwendige Nadelungsverfahren einsetzen zu müssen, das beim Verbinden von auch im Randverschlussbereich silikonisierten Verpackungsfolien angewendet werden muss.

Trotz der durch die Verbesserung des Verpackungsmaterials, insbesondere durch die Anordnung der Release-Beschichtung erzielten produktionstechnischen Vorteile bei der Einzelverpackung von auf einer Seite klebefähig ausgerüsteten Hygieneprodukten, ergab sich kein Vorteil im Hinblick auf eine Verhinderung der Geräuschereignisse beim Öffnen einer solchen Verpackung, das für viele Anwender, wie bereits erwähnt, zu einem unangenehmen und für den Verbraucher peinlichen Ereignis führt.

Aufgabe der vorliegenden Erfindung war daher, eine geräuscharm zu öffnende Einschlagverpackung für ein einseitig klebendes Hygieneprodukt zur Verfügung zu stellen.

Diese Aufgabe wird erfindungsgemäß gelöst durch die Bereitstellung einer geräuscharm zu öffnenden Einschlagverpackung (5) gemäß Anspruch 1.

Unter dem Begriff "geräuscharmes Öffnen" wird erfindungsgemäß das beim Öffnen der Verpackung entstehende und als energieäquivalenter Dauerschaltpegel L_{A}eq ≤ 60 dB ausgewertete Geräusch verstanden. Die Definition des energieäquivalenten Dauerschaltpegels L_{A}eq basiert auf der Messung des Schalldruckpegels. Der Schalldruckpegel gibt an, wie laut ein Geräusch zu einem gewissen Zeitpunkt ist, und weist im Allgemeinen zeitliche Schwankungen auf. Mit dem Schalldruckpegel variiert auch die mit dem Schall transportierte Energie. Der energieäquivalente Dauerschallpegel wird so gewählt, dass er - als konstanter Schalldruckpegel betrachtet - den gleichen Energieinhalt transportieren würde. Für die Festlegung von Lärmgrenzwerten wird vorzugsweise der energieäquivalente Dauerschallpegel herangezogen (vgl. Veröffentlichung des österreichischen Umweltbundesamts).

Erfindungsgemäß wird erreicht, dass der so definierte energieäquivalente Dauerschallpegel beim Öffnen von erfindungsgemäßen Einschlagverpackungen für flächenförmige, einseitig mit einem Kleber versehene Hygieneprodukte gegenüber den am Markt bereits befindlichen bzw. bereits bekannten Einschlagverpackungen mit diesen darin verpackten Produkten um mindestens 10 % vermindert wird, so dass das Geräusch beim Öffnen der erfindungsgemäßen Verpackung einem leisen bis normalen Gespräch mit ≤ 60 dB statt einem lauten Gespräch von ungefähr 70 dB entspricht (vgl. Veröffentlichung des österreichischen Umweltbundesamts unter www.umweltbundesamt.at/umweltsituation/laerm/schalldruckpegel).

Das Verpackungsmaterial, aus dem die erfindungsgemäße, geräuscharm zu öffnende Einschlagverpackung hergestellt wird, ist ein bahnförmiges Verpackungsmaterial, das aus einer Kunststofffolie, die eine Mono- oder Mehrschichtfolie sein kann, aufgebaut ist.

Sofern die Kunststofffolie eine Monofolie ist, besteht sie aus einer heißsiegelbaren Schicht a), wie in den Ansprüchen definiert.

Sofern das erfindungsgemäße Verpackungsmaterial aus einer Mehrschichtfolie aufgebaut ist, weist deren Siegelschicht a) eine Zusammensetzung entsprechend der Monofolie auf.

Erfindungsgemäß werden Mischungen aus LDPE und LLDPE, die überwiegend aus LDPE bestehen, als Mischungskomponente für das Polybutylen zur Herstellung der Schicht a) eingesetzt.

Mit "LDPE" werden Polyethylene mit niedriger Dichte bezeichnet, die eine Dichte im Bereich von 0,86-0,93 g/cm³ aufweisen und sich durch einen hohen Verzweigungsgrad der Moleküle auszeichnen.

Mit "LLDPE" werden lineare Ethylen-Copolymere niedriger Dichte bezeichnet, welche durch das Vorhandensein einer linearen Hauptkette mit daran befindlichen Seitenketten gekennzeichnet sind und eine Dichte im Bereich von 0,86 und 0,94 g/cm³ aufweisen.

Als übliche Hilfsstoffe, mit der die Schicht a) dotiert werden kann, kommen Farbpigmente und/oder Füllstoffe, wie z. B. Titandioxid oder Kreide in Frage, die vorzugsweise bis zu 20 Gew.-% bezogen auf das Gesamtgewicht der Schicht a), besonders bevorzugt von 3 - 18 Gew.-% bezogen auf das Gesamtgewicht der Schicht a), zur Mattierung und/oder Einfärbung in der Schicht a) vorliegen können.

Sofern das Verpackungsmaterial, das zur Herstellung der erfindungsgemäßen Einschlagverpackung verwendet wird, eine Mehrschichtfolie ist, so kann diese Mehrschichtkunststofffolie neben der Schicht a) eine angrenzende Schicht b) aufweisen, aus Polyethylen mit einer Dichte von 0,88 - 0,94 g/cm³. Vorzugsweise können zur Herstellung der Schicht b) Mischungen von LDPE und LLDPE eingesetzt werden, die ebenso wie die Schicht a) ggf. übliche Hilfsstoffe, vorzugsweise die vorstehend aufgeführten, aufweisen kann. Die Mischungen aus LDPE und LLDPE weisen vorzugsweise 40 - 80 Gew.-% LDPE und 60 - 20 Gew.-% LLDPE, jeweils bezogen auf die Gesamtmischung, auf.

In einer weiteren bevorzugten Ausführungsform der vorliegenden Erfindung, ist das Verpackungsmaterial, aus der die erfindungsgemäße Einschlagverpackung hergestellt wird, eine Mehrschichtfolie, die eine Schicht b) aufweist, auf deren Oberflächen jeweils eine Schicht a) angeordnet ist.

Ggf. kann eine Oberfläche einer solchen Mehrschichtfolie auch mit einer Vliesbahn verbunden sein.

Die erfindungsgemäße Einschlagverpackung kann auch nur aus einer Mono-Kunststofffolie, d. h. der Schicht a), oder eine zweischichtige Kunststofffolie aus der Schicht a) und b), die auf jeweils einer Oberfläche mit einer Vliesbahn verbunden ist, bestehen.

Eine solche Vliesbahn stellt bevorzugt die Außenseite der erfindungsgemäßen Einschlagverpackung dar.

Vorzugsweise liegt die Schichtdicke der Schicht a) im Bereich von 3 - 10 µm und die Schichtdicke der Schicht b) im Bereich von 5 - 20 µm.

Die erfindungsgemäß zum Einsatz kommende Mono- oder Mehrschichtkunststofffolie weist auf der Schicht a) zumindest in den Abschnitten, die als Verpackungsmaterial der mit einem Kleber versehenen Seite des verpackten Hygieneprodukts (4) zugewandt und damit lösbar verbunden sind, eine Release-Beschichtung (2) und im Übrigen zumindest in den Teilbereichen, die zu streifenförmigen oder linearen Randverschlüssen bei der Herstellung der Einschlagverpackung (5) verbunden werden, keine Release-Beschichtung auf.

Dementsprechend sind die Abschnitte der Schicht a), die mit einer Release-Beschichtung versehen sind, vorzugsweise streifenförmig in Folienbahnrichtung und vorzugsweise entsprechend der Anordnung der klebefähigen Abschnitte des zu verpackenden Hygieneproduktes angeordnet.

Vorzugsweise basiert die Release-Beschichtung auf einem Polysiloxan, das ausgehärtet ist.

Die erfindungsgemäße Einschlagverpackung wird hergestellt, indem das zu verpackende Hygieneprodukt, zumindest mit seinen klebefähigen Abschnitten, auf die mit Release-Beschichtung ausgerüsteten Abschnitten der Kunststofffolie als Verpackungsmaterial in Kontakt gebracht wird. Das in Abschnitten der zum Einsatz kommenden Kunststofffolie vorliegende Verpackungsmaterial, das jeweils einen seitlichen Abschnitt, der nicht von dem zu verpackenden Hygieneprodukt bedeckt ist, aufweist, wird ggf. zusammen mit dem Hygieneprodukt durch Faltung dieser beiden seitlichen Abschnitte auf derselben Oberfläche des zu verpackenden Hygieneprodukts gegeneinander und ggf. überlappend so angeordnet, dass die Randstreifen des Verpackungsmaterialsabschnitts übereiander liegen. Um diese Randstreifen dauerhaft, aber aufreißbar miteinander zu verbinden, werden sie durch Heißsiegelung, Verklebung, Verkrümperung oder Nadelung in parallel verlaufende streifenförmige oder einfach oder mehrfach linienförmige, seitliche Randverschlüsse verbunden. Üblicherweise wird der Bereich, der ggf. überlappenden, gefalteten Seitenabschnitte, der quer zur Folienbahnrichtung bzw. zu den seitlichen Randverschlüssen verläuft, nicht verschlossen, damit das Öffnen zum Entnehmen des Hygieneproduktes einfacher erfolgen kann.

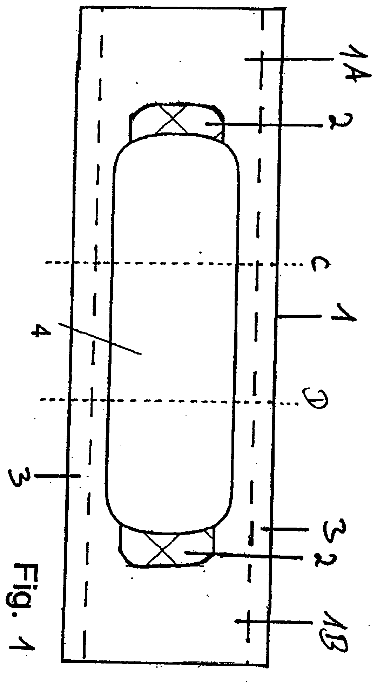

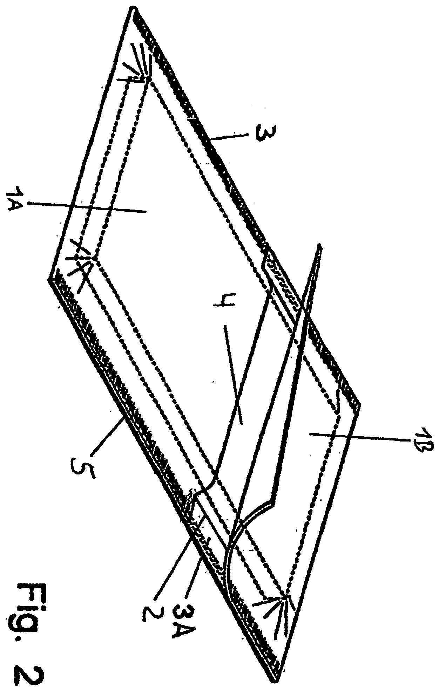

Eine erfindungsgemäße Einschlagverpackung vor der Faltung der Seitenbereiche ist in Figur 1 dargestellt, während in Figur 2 die fertige Einschlagverpackung schematisch dargestellt ist.

Die Figur 1 beschreibt einen Folienbahn-Abschnitt eines erfindungsgemäß zum Einsatz kommenden Verpackungsmaterial mit einem mittleren Längsstreifen mit Release-Beschichtung, der etwas länger als das klebefähige Hygieneprodukt zur Veranschaulichung dargestellt ist.

Die Figur 2 ist eine aus dem Folienbahn-Abschnitt des erfindungsgemäß zum Einsatz kommenden Verpackungsmaterials hergestellte Einschlagverpackung 5.

Mit 1 wird in Figur 1 ein Abschnitt einer Mehrschichtkunststofffolienbahn bezeichnet, die als Verpackungsmaterial zur Herstellung einer erfindungsgemäßen Einschlagverpackung 5 Verwendung findet. Dieser Abschnitt ist mit einer Release-Beschichtung 2 versehen, auf dem ein flächenförmiges Hygieneprodukt 4 aufgelegt ist, das in der Einschlagverpackung 5 verpackt werden soll. Das Hygieneprodukt 4 ist eine Damenbinde, die auf ihrer Unterseite mit einem Haftkleber versehen ist (nicht dargestellt), der auf der Release-Beschichtung 2 lösbar verbunden ist und ohne Verlust der Klebkraft haftet. Zwei seitliche Abschnitte 1A und 1B des mit 1 bezeichneten Abschnitts der Verpackungsmaterialbahn, die keine Release-Beschichtung bis auf den Bereich 2 aufweist, werden zum Verpacken der Damenbinde mit dieser entlang der Faltlinien C bzw. D (als Punkt-Linie dargestellt) gefaltet und die übereinanderliegenden Randstreifen 3, die durch Strichlierung in Figur 1 angedeutet sind, miteinander dauerhaft, aber aufreißbar verbunden. Diese parallel verlaufenden, streifenförmigen oder linienförmigen, seitlichen Randverschlüsse können durch Heißsiegelung, Verklebung, Verkrümperung oder Nadelung der übereinander liegenden Randbereiche der Schicht a) erhalten werden. Besonders bevorzugt wird die Verbindung der Randbereiche zu streifenförmigen oder einfach oder mehrfach linienförmigen Randverschlüssen durch Heißsiegelung mit Hilfe vorzugsweise bekannter Heißsiegelvorrichtungen zum Versiegeln von Verpackungsfolien durchgeführt.

In Figur 2 ist eine so hergestellte Einschlagverpackung 5 dargestellt, die durch Faltung der Verpackungsabschnitte 1A und 1B zusammen mit der zu verpackenden Damenbinde (Hygieneprodukt) 4 und Versiegelung der übereinanderliegenden Randbereiche der Schicht a) der als Verpackungsmaterial verwendeten Kunststofffolie erhalten wurde. Der Randverschluss der Einschlagverpackung ist zu Beginn des Öffnens der Einschlagverpackung in Bereich 3A zum Teil bereits geöffnet und liegt im Bereich 3 noch als streifenförmige Randversiegelung der erfindungsgemäßen Einschlagverpackung vor.

Die erfindungsgemäßen Verpackungen, in Form von Einschlagverpackungen, eignen sich zum Einzelverpacken von flächenförmigen, einseitig mit einem Kleber, vorzugsweise Haftkleber, versehenen Hygieneprodukte, vorzugsweise als Einzelverpackung einer Damenbinde, einer Slipeinlage oder eines flächenförmigen Inkontinenzproduktes, wobei das Hygieneprodukt vorzugsweise gefaltet in der Verpackung vorliegt.

Ein weiterer Gegenstand der vorliegenden Erfindung ist daher eine erfindungsgemäße Einzelverpackung, in Form einer Einschlagverpackung, in der eine Damenbinde, eine Slipeinlage oder ein flächenförmiges Inkontinenzprodukt, vorzugsweise gefaltet, verpackt ist.

### Bezugszeichenliste

| | |
|---|---|
| 1 | Abschnitt der erfindungsgemäßen Kunststofffolienbahn als Verpackungsmaterial |
| 1A 1B | 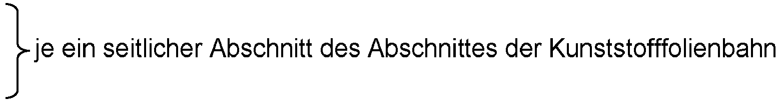 |
| 2 | Release-Beschichtung |
| 3 | verbundene Randstreifen / Siegelnaht |
| 3A | nicht verbundener Abschnitt der Randstreifen 3 |
| 4 | Hygieneprodukt |
| 5 | Einschlagverpackung |
| C D | 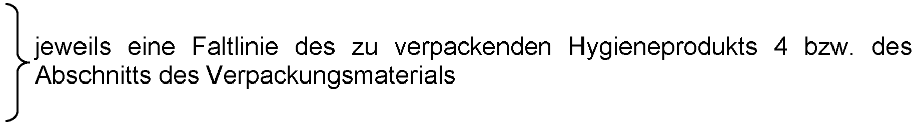 |

### Geräusch-Messung

### I. Mess-Bedingungen

| | |
|---|---|
| Messgerät: | 2250-L von Brüel & Kjaer |
| Software: | BZ7133 Version 2.0.2 |
| Raum: | Schalldichter Raum 1 m × 1m Grundfläche und 2 m Höhe ausgestattet mit schallschluckenden Vlies- bzw. Gewebe-beklebten Wänden. |
| Abstand zwischen Messgerät und Öffnungsvorgang | 20 cm |
| Öffnungsgeschwindigkeit: | 3 cm/s |

### II. Beschreibung des Messvorganges

Jeweils 10 Einschlagverpackungen aus identischen, nachstehend aufgeführten Verpackungsfolien mit jeweils identischer Release-Beschichtung und jeweils einer identischen, darin verpackten, gefalteten Damenbinde werden in einem schalldichten Raum (Abmaße: B/L/H = 1m/1m/2m) im Abstand von 20 cm zum Messgerät mit gleichbleibender Öffnungsgeschwindigkeit von derselben Person geöffnet.

| | |
|---|---|
| Gemessen werden: | L_{Aeq} in der Einheit [dB] |

Ermittelt wird der höchste gemessene L_{Aeq} in der Einheit [dB] während der Öffnung der Damenbindenverpackung. Der L_{Aeq} [dB] ist der äquivalente Dauerschallpegel für eine Mittelungszeit T. Die Mittelungszeit T wurde mit 1/10 Sekunde definiert.

### Beispiele und Vergleichsbeispiele

### I. Polymere und Hilfsstoffe:

| | |
|---|---|
| LDPE (Polyethylen mit niedriger Dichte) | Lupolen 2420 F der Firma Basell |
| Kreidebatch (72 Gew.-% Kreide in LDPE) | Multibatch ME 50009 der Firma Multibase |
| Polybutylen | Polybuten 8640 M der Firma Basell |
| LLDPE (lineares Polyethylen mit niedriger Dichte ) | Innovex LL 6910 der Firma BP |
| Farbmasterbatch (70 Gew.-% TiO₂ in LDPE) | Remafin Weiss RCL der Firma Clariant |

### II. Herstellung der Einschlagverpackung

Aus den in der nachstehenden Tabelle aufgeführten Folien als Verpackungsmaterial, die jeweils eine Release-Beschichtung aus ausgehärtenden Polysiloxan im jeweils klebefähigen Bereich der zu verpackenden Damenbinde aufwiesen, wurden, wie vorstehend beschrieben, Einschlagverpackungen hergestellt, deren 0,5 mm breiten Randverschlüsse durch Versieglung der übereinander liegenden Folienabschnitte der Schicht a) mit Hilfe eines Siegelwerkzeugs mit beheizbaren Siegelbalken unter einer Druckanwendung von 4,5 bar bei einer Temperatur von 105°C und eine Siegeldauer von 0,5 sec hergestellt wurden. Die % Angaben sind immer Gew.-%.

### III. Folienaufbau

| | **Folienaufbau des Verpackungsmaterials** | **Randverschlüsse der Verpackung (0,5 mm)** |
|---|---|---|
| **Vergleichsbeispiele:** | | |
| Vergleichsbeispiel 1 | LDPE (Monofolie, Dicke 25 µm) | versiegelt |
| Vergleichsbeispiel 2 | 56% LDPE + 22,5% Kreidebatch + 19,5 LLDPE + 2% Farbmasterbatch (Monofolie, Dicke 25 µm) | versiegelt |

| Beispiele: | | |
|---|---|---|
| Beispiel 1 | 85% LDPE + 15% Polybutylen (Monofolie, Dicke 25 µm) | versiegelt |
| Beispiel 2 | 39 % LDPE + 19 % Kreidebatch + 25% LLDPE + 2% Farbmasterbatch + 15% Polybutylen (Monofolie, Dicke 25 µm) | versiegelt |
| Beispiel 3 | Schicht a [Dicke: 5µm]: | versiegelt |
| | 39 % LDPE + 19 % Kreidebatch + 25% LLDPE + 2% Farbmasterbatch + 15% Polybutylen | |
| | Schicht b [Dicke:15µm]: | |
| | 56% LDPE + 22,5% Kreidebatch + 19,5 LLDPE + 2% Farbmasterbatch | |
| | Schicht a [Dicke: 5µm]: | |
| | 39 % LDPE + 19 % Kreidebatch + 25% LLDPE + 2% Farbmasterbatch + 15% Polybutylen | |
| | (3-schichtige Folie) | |

### IV. Geräuschmessresultate beim Öffnen

| **Messung** | **L_{Aeq} [dB] Vergleichsbeispiel 1** | **L_{Aeq} [dB] Vergleichsbeispiel 2** | **L_{Aeq} [dB]** Vergleichsbeispiel 3 | **L_{Aeq} [dB] Beispiel 2** | **L_{Aeq} [dB] Beispiel 3** |
|---|---|---|---|---|---|
| 1 | > 70 | > 70 | 59,9 | 59,8 | 60 |
| 2 | > 70 | > 70 | 60,1 | 59,7 | 59,1 |
| 3 | > 70 | > 70 | 58,3 | 57,9 | 58,5 |
| 4 | > 70 | > 70 | 60,2 | 59 | 58,7 |
| 5 | > 70 | > 70 | 59,4 | 60,1 | 59,5 |
| 6 | > 70 | > 70 | 58,7 | 58,2 | 58,1 |
| 7 | > 70 | > 70 | 59,9 | 59,1 | 58,2 |
| 8 | > 70 | > 70 | 58,2 | 57,7 | 58 |
| 9 | > 70 | > 70 | 60 | 58,2 | 57,6 |
| 10 | > 70 | > 70 | 58,9 | 58,7 | 58,4 |
| Mittelwert: [dB] | > 70 | > 70 | 59,36 | 58,84 | 58,61 |

Die Geräuschmessungen wurden gemäß der vorstehend angegebenen Methode durchgeführt. Beispiel 1 ist kein Beispiel der Erfindung, sondern auf zu fassen als Vergleichsbeispiel 3.

### Resultate:

Die Messungen zeigen, dass die erfindungsgemäßen Verpackungen geräuschärmer zu öffnen sind als Verpackungen, deren Verpackungsmaterial keine erfindungsgemäße Schicht a) aufweist. Außerdem können die Verpackungen gemäß Vergleichsbeispiele jeweils nur unter Zerstörung des Verpackungsmaterials geöffnet werden.

## Patentansprüche

1. Eine geräuscharm zu öffnende Einschlagverpackung (5) für ein flächenförmiges, einseitig mit einem Kleber versehenes Hygieneprodukt (4), die auf einem bahnförmigen Verpackungsmaterial (1) aus einer Kunststofffolie basiert, und wobei das beim Öffnen der Einschlagverpackung (5) entstehende Geräusch einem energieäquivalenten Dauerschallpegel LAeq ≤ 60 dB entspricht, gemessen nach der in der Beschreibung angegebenen Methode,
**dadurch gekennzeichnet, dass** die Kunststofffolie
a) wenigstens eine heißsiegelbare Schicht bestehend aus einer Mischung aus LDPE und LLDPE, die überwiegend aus LDPE besteht, und aus 3 bis höchstens 30 Gew.-%, bezogen auf das Gesamtgewicht der Schicht a), Polybutylen und ggf. bis zu 20 Gew.-% bezogen auf das Gesamtgewicht der Schicht a) Farbpigmenten und/oder Füllstoffen; und
b) ggf. eine Schicht aus Polyethylen mit einer Dichte von 0,88 - 0,94 g/cm³ umfasst,
wobei die Schicht a) zumindest in den Abschnitten, die den mit dem Kleber versehenen Bereichen des Hygieneproduktes (4) zugewandt und damit lösbar verbunden sind, eine Release-Beschichtung (2) und im Übrigen zumindest in den zu streifenförmigen Randverschlüssen durch Heißsiegelung verbundenen Teilbereichen (3) der Einschlagverpackung keine Release-Beschichtung aufweist.

2. Eine Einschlagverpackung (5) nach Anspruch 1, **dadurch gekennzeichnet, dass** die Schicht a) der Kunststofffolie 5 - 20 Gew.-%, bezogen auf das Gesamtgewicht der Schicht a), Polybutylen aufweist.

3. Eine Einschlagverpackung (5) nach Anspruch 1 oder 2, **dadurch gekennzeichnet, dass** die Kunststofffolie aus einer Schicht b), die als Außenschichten jeweils eine Schicht a) aufweist, besteht.

4. Eine Einschlagverpackung (5) nach Anspruch 1 oder 2, **dadurch gekennzeichnet, dass** die Einschlagverpackung aus einer Mono-Kunststofffolie aus der Schicht a) besteht, oder aus eine zweischichtige Kunststofffolie aus der Schicht a) und b) besteht, die auf jeweils einer Oberfläche mit einer Vliesbahn verbunden ist.

5. Eine Einschlagverpackung (5) nach einem der Ansprüche 1 - 4, **dadurch gekennzeichnet, dass** die Polyethylen-Komponente der Schicht b) aus einer Mischung von LDPE und LLDPE besteht.

6. Eine Einschlagverpackung (5) nach einem der Ansprüche 1 - 5, **dadurch gekennzeichnet, dass** die mit Release-Beschichtungen (2) ausgerüsteten Abschnitte auf der Schicht a), die mit den klebefähigen Abschnitten des verpackten, vorzugsweise gefalteten Hygieneproduktes (4) lösbar verbunden sind, auf einer ausgehärteten Polysiloxanbeschichtung basieren.

7. Eine Einschlagverpackung (5) nach einem Ansprüche 1 - 6, **dadurch gekennzeichnet, dass** die mit der Release-Beschichtung (2) ausgerüsteten Abschnitte der Schicht a) streifenförmig und/oder entsprechend der Anordnung der klebefähigen Abschnitte des verpackten Hygieneproduktes (4) angeordnet sind.

8. Eine Einschlagverpackung (5) nach einem der Ansprüche 1 - 7, **dadurch gekennzeichnet, dass** die parallel verlaufenden, streifenförmigen oder parallel verlaufenden, einfach oder mehrfach linienförmigen Randverschlüsse der Einschlagverpackung jeweils auf einer dauerhaften Verbindung durch Heißsiegelung der im Randbereich übereinanderliegenden, keine Release-Beschichtung aufweisenden Bereiche (3) der Schicht a) beruhen, die durch jeweilige Faltung zweier seitlicher Abschnitte des Verpackungsmaterials gegeneinander und ggf. überlappend auf derselben Oberfläche des Hygieneprodukts (4) beim Verpacken des vorzugsweise dabei mit gefalteten Hygieneproduktes (4) erhalten wurden.

9. Eine Einschlagverpackung (5) nach einem der Ansprüche 1 - 8, **dadurch gekennzeichnet, dass** das verpackte, vorzugsweise gefaltete Hygieneprodukt (4) eine Damenbinde, eine Slipeinlage oder ein flächenförmiges Inkontinenzprodukt ist.

10. Eine Einschlagverpackung (5) nach einem der Ansprüche 1 - 9, **dadurch gekennzeichnet, dass** die Einschlagverpackung (5) auf einer mattierten ggf. eingefärbten Kunststofffolie beruht.

11. Eine Einschlagverpackung (5) nach einem der Ansprüche 1 - 10, **dadurch gekennzeichnet, dass** sie bedruckt ist.

## Claims

1. A quiet-opening wrapper (5) for a sheet-like hygiene product (4) provided with an adhesive on one side, which product is based on a web-like packaging material (1) made of a plastic film, and wherein the noise generated when opening the wrapper (5) corresponds to an energy-equivalent continuous sound level LAeq ≤ 60 dB, measured according to the method specified in the description,
**characterized in that** the plastic film comprises
a) at least one heat-sealable layer consisting of a mixture of LDPE and LLDPE, which consists predominantly of LDPE, and of 3 to a maximum of 30% by weight, based on the total weight of layer a), polybutylene and optionally up to 20% by weight, based on the total weight of layer a), color pigments and/or fillers; and
b) if necessary, a layer of polyethylene with a density of 0.88 - 0.94 g/cm³,
wherein layer a) has a release coating (2) at least in the portions which face the regions of the hygiene product (4) provided with the adhesive and are detachably connected thereto, and otherwise has no release coating at least in the partial regions (3) of the wrapper which are connected by heat sealing to form strip-shaped edge closures.

2. The wrapper (5) according to claim 1, **characterized in that** layer a) of the plastic film contains 5-20 wt.% polybutylene, based on the total weight of layer a).

3. The wrapper (5) according to claim 1 or 2, **characterized in that** the plastic film consists of a layer b), which respectively has a layer a) as the outer layers.

4. The wrapper (5) according to claim 1 or 2, **characterized in that** the wrapper is made of a mono-plastic film from layer a) or a two-layer plastic film from layer a) and b), which are connected to a nonwoven web on a respective surface.

5. The wrapper (5) according to any one of claims 1 - 4, **characterized in that** the polyethylene component of layer b) consists of a mixture of LDPE and LLDPE.

6. The wrapper (5) according to any one of claims 1-5, **characterized in that** the portions on layer a) provided with release coatings (2) and which are detachably connected to the adhesive portions of the packaged, preferably folded hygiene product (4), are based on a cured polysiloxane coating.

7. The wrapper (5) according to any one of claims 1-6, **characterized in that** the portions of layer a) provided with the release coating (2) are arranged in strip form and/or in accordance with the arrangement of the adhesive portions of the packaged hygiene product (4).

8. The wrapper (5) according to any one of claims 1 - 7, **characterized in that** the parallel, strip-shaped or parallel, single or multiple line-shaped edge closures of the wrapper are respectively based on a permanent connection by heat sealing of the regions (3) of layer a) which lie one above the other in the edge region and have no release coating and which were obtained by folding two lateral portions of the packaging material against each other and optionally overlapping on the same surface of the hygiene product (4) when packaging the hygiene product (4), which is preferably folded at the same time.

9. The wrapper (5) according to any one of claims 1 - 8, **characterized in that** the packaged, preferably folded hygiene product (4) is a sanitary napkin, a panty liner or a sheet-like incontinence product.

10. The wrapper (5) according to any one of claims 1 - 9, **characterized in that** the wrapper (5) is based on a matte, optionally colored plastic film.

11. The wrapper (5) according to any one of claims 1 - 10, **characterized in that** it is printed.

## Revendications

1. Enveloppe (5) pouvant être ouverte avec peu de bruit pour un produit d'hygiène (4) plat pourvu d'un adhésif sur une face, qui est à base d'un matériau d'emballage en forme de bande (1) constitué d'un film plastique, et dans laquelle le bruit produit lors de l'ouverture de l'enveloppe (5) correspond à un niveau sonore continu équivalent en énergie LAeq ≤ 60 dB, mesuré selon la méthode indiquée dans la description,
**caractérisée en ce que** le film plastique comprend
a) au moins une couche thermoscellable constituée d'un mélange de LDPE et de LLDPE, constitué principalement de LDPE, et de 3 à maximum 30 % en poids de polybutylène, par rapport au poids total de la couche a), et éventuellement jusqu'à 20 % en poids de pigments colorés et/ou de charges, par rapport au poids total de la couche a) ; et
b) éventuellement, une couche de polyéthylène d'une densité de 0,88 à 0,94 g/cm³ ,
dans laquelle la couche a) présente un revêtement antiadhésif (2) au moins dans les zones qui font face aux zones du produit d'hygiène (4) pourvues de l'adhésif et qui y sont reliées de manière amovible, et ne présente, sinon, pas de revêtement antiadhésif au moins dans les zones partielles (3) de l'enveloppe, reliées aux fermetures de bord en forme de bandes par thermoscellage.

2. Enveloppe (5) selon la revendication 1, **caractérisée en ce que** la couche a) du film plastique contient 5 à 20 % en poids de polybutylène, par rapport au poids total de la couche a).

3. Enveloppe (5) selon la revendication 1 ou 2, **caractérisée en ce que** le film plastique est constitué d'une couche b), dont chacune présente une couche a) comme couches extérieures.

4. Enveloppe (5) selon la revendication 1 ou 2, **caractérisée en ce que** la couche a) ou une couche composite de couches a) et b) est reliée à une bande non tissée adjacente à la couche b) en tant que couche de support.

5. Enveloppe (5) selon l'une des revendications 1 à 4, **caractérisée en ce que** le composant polyéthylène de la couche b) est constitué d'un mélange de LDPE et de LLDPE.

6. Enveloppe (5) selon l'une des revendications 1 à 5, **caractérisée en ce que** les sections pourvues de revêtements antiadhésifs (2) sur la couche a), qui sont reliées de manière amovible avec les sections adhésives du produit d'hygiène (4) emballé, de préférence plié, sont à base d'un revêtement en polysiloxane durci.

7. Enveloppe (5) selon l'une des revendications 1 à 6, **caractérisée en ce que** les sections de la couche a) pourvues du revêtement antiadhésif (2) sont en forme de bande et/ou sont disposées de préférence de manière correspondante à la disposition des sections adhésives du produit d'hygiène (4) emballé.

8. Enveloppe (5) selon l'une des revendications 1 à 7, **caractérisée en ce que** les fermetures de bord, parallèles, en forme de bande ou parallèles, simples ou multiples en forme de ligne, de l'enveloppe sont chacune basées sur une liaison permanente par thermoscellage des zones (3) de la couche a) superposées dans la zone de bord et ne présentant pas de revêtement antiadhésif, lesquelles ont été obtenues par pliage respectif de deux sections latérales du matériau d'emballage l'une contre l'autre et se chevauchant éventuellement sur la même surface du produit d'hygiène (4) lors de l'emballage du produit d'hygiène (4), de préférence plié en même temps.

9. Enveloppe (5) selon l'une des revendications 1 à 8, **caractérisée en ce que** le produit d'hygiène (4) emballé, de préférence plié, est une serviette hygiénique, un protège-slip ou un produit plat pour l'incontinence.

10. Enveloppe (5) selon l'une des revendications 1 à 9, **caractérisée en ce que** l'enveloppe (5) est basée sur un film plastique mat, éventuellement coloré.

11. Enveloppe (5) selon l'une des revendications 1 à 10, **caractérisée en ce qu'**elle est imprimée.
